# EUROPÄISCHE PATENTSCHRIFT

(11) **EP 1 608 335 B1**
(45) Veröffentlichungstag und Bekanntmachung des Hinweises auf die Patenterteilung: **05.12.2007**
(21) Anmeldenummer: 04718201.9
(22) Anmeldetag: 06.03.2004
(51) Int. Cl.: A61K 9/08

(54) **KONTROLLIERTES FREISETZUNGSSYSTEM ENTHALTEND SACCHAROSEACETATISOBUTYRAT**
CONTROLLED RELEASE SYSTEM CONTAINING SACCHAROSE ACETATE ISOBUTYRATE
SYSTEME DE LIBERATION CONTROLEE CONTENANT DE L'ACETATE ISOBUTYRATE DE SACCHAROSE

(30) Priorität: 20.03.2003 DE 10312346
(43) Veröffentlichungstag der Anmeldung: 28.12.2005
(73) Patentinhaber: Bayer HealthCare AG, 51368 Leverkusen (DE)
(72) Erfinder: FRAATZ, Kristine, 51399 Burscheid (DE); MERTIN, Dirk, 40764 Langenfeld (DE); HEEP, Iris, 50859 Köln (DE)
(86) Internationale Anmeldenummer: PCT/EP2004/002318
(87) Internationale Veröffentlichungsnummer: WO 2004/082658

(56) Entgegenhaltungen:
- WO-A-01/15734
- WO-A-20/04032898
- US-A- 5 747 058

## Beschreibung

Die Erfindung betrifft ein System zur kontrollierten Freisetzung arzneilich wirksamer Substanzen, welches Saccharoseacetatisobutyrat (SAIB) sowie ein weiteres Solvens enthält.

Es gibt eine umfangreiche Forschung auf dem Gebiet der kontrollierten Freisetzung von pharmazeutischen Wirkstoffen mittels bioabbaubarer Systeme. Sie haben den Vorteil, dass sie nicht nach Wirkstoffentleerung aus dem Körper entfernt werden müssen.

Einfach und somit kostengünstig sind bioabbaubare Systeme wie sie in WO 96/39995 (bzw. den korrespondierenden US-Patenten US 5747058 und US 5968542) beschrieben werden. Hierbei dient eine hochviskose Flüssigkeit, vorzugsweise Saccharoseacetatisobutyrat (SAIB) als Wirkstoffträger. Aufgrund der hohen Viskosität kann SAIB allerdings in der Regel nur nach Verdünnung mit einem geeigneten Lösungsmittel parenteral appliziert werden. Als geeignete Lösungsmittel werden in den Schriften Ethanol, Dimethylsulfoxid, Ethyllactat, Ethylacetat, Benzylalkohol, Triacetin, N-Methylpyrrolidon, Propylencarbonat und Glykofurol genannt. Bislang für diesen Zweck nicht vorgeschlagene Solventien sind Glycerinformal und Solketal (Isopropylidenglycerol).

Allerdings beschreibt WO 01/15734 flüssige Zubereitungen enthaltend einen hochviskosen flüssigen Träger, wobei SAIB bevorzugt und in mehreren Beispielen offenbart wird, sowie Lösungsmittel, wobei unter anderem auch 2,2-Dimethyl-1,3-dioxolan-4-methanol genannt wird.

Es wurde gefunden, dass Glycerinformal und Solketal mit SAIB mischbar sind und sich ausgezeichnet zur Herstellung bioabbaubarer pharmazeutischer Systeme zur kontrollierten Freisetzung arzneilich wirksamer Substanzen eignen. Der Zusatz von Glycerinformal und/oder Solketal verbessert das Lösungsvermögen für viele Wirkstoffe, insbesondere für Fluorchinolone oder Analgetika wie z.B. Flupirtin. Durch den Einsatz von Glycerinformal und Solketal zusammen mit SAIB können somit bioabbaubare pharmazeutische Systeme zur retardierten Wirkstoff-Freisetzung erhalten werden.

Gegenstand der Erfindung ist daher:
1. Arzneimittel enthaltend:
   (a) einen pharmazeutisch wirksamen Bestandteil
   (b) Saccharoseacetatisobutyrat (SAIB)
   (c) als Solvens Glycerinformal oder Isopropylidenglycerol oder ein Gemisch davon sowie als Kosolvenz einen pharmazeutisch verträglichen Alkohol.

Das Solvens wird typischerweise in einem Anteil von mindestens 5 Gew.-%, bevorzugt mindestens 10 Gew.-% besonders bevorzugt mindestens 15 Gew.-% eingesetzt, üblicherweise im Bereich 5 bis 80 Gew.-%, bevorzugt 10 bis 70 Gew.-%, insbesondere 15 bis 60 Gew.-%, wobei die Angaben in Gew.-% jeweils bezogen sind auf das Gesamtgewicht der Formulierung.

SAIB wird üblicherweise in Konzentrationen von maximal 80 Gew.-%, bevorzugt maximal 75 Gew.-% eingesetzt, beispielsweise im Bereich 5 bis 80 Gew.-%, bevorzugt 10 bis 75 Gew.-% jeweils bezogen auf das Gesamtgewicht der Formulierung. Durch Variation der SAIB-Konzentration lassen sich verschiedene Freisetzungscharakteristika erzielen:

Gemäß einer Ausführungsform betrifft die Erfindung Depotformulierungen mit lang anhaltender Freisetzung; diese enthalten üblicherweise 50 bis 80 Gew.-%, bevorzugt 60 bis 75 Gew.-% SAIB, bezogen auf das Gesamtgewicht der Formulierung. Aufgrund des hohen SAIB-Anteils ist in solchen Formulierungen der Anteil an Solvens zwangsläufig geringer, er ist in der Regel kleiner als 45 Gew.-%, bevorzugt kleiner als 40 Gew.-%, besonders bevorzugt kleiner als 30 Gew.-%.

Gemäß einer weiteren Ausführungsform betrifft die Erfindung Retardformulierungen mit nur leicht verzögerter Freisetzung des Wirkstoffs; diese enthalten üblicherweise 10 bis 50 Gew.-%, bevorzugt 20 bis 40 Gew.-% SAIB. Solche Retardformulierungen sind aufgrund des etwas langsameren Anflutens und eines geringeren Bursts des Wirkstoffs oft besser verträglich als Formulierungen, bei denen die Freisetzung des Wirkstoffs nicht verzögert wird.

Als pharmazeutisch wirksamer Bestandteil kommt ein pharmazeutischer Wirkstoff oder eine Kombination mehrerer Wirkstoffe in Frage. Bevorzugt werden in den erfindungsgemäßen Arzneimitteln als pharmazeutische Wirkstoffe Fluorchinolone oder Analgetika wie z.B. Flupirtin eingesetzt. Fluorchinolone sind unter anderem Verbindungen, wie sie in folgenden Dokumenten offenbart sind: US 4 670 444 (Bayer AG), US 4 472 405 (Riker Labs), US 4 730 000 (Abbott), US 4 861 779 (Pfizer), US 4 382 892 (Daüchi), US 4 704 459 (Toyama), als konkrete Beispiele seien genannt: Benofloxacin, Binfloxacin, Cinoxacin, Ciprofloxacin, Danofloxacin, Difloxacin, Enoxacin, Enrofloxacin, Fleroxacin, Ibafloxacin, Levofloxacin, Lomefloxacin, Marbofloxacin, Moxifloxacin, Norfloxacin, Ofloxacin, Orbifloxacin, Pefloxacin, Pipemidsäure, Temafloxacin, Tosufloxacin, Sarafloxacin, Sparfloxacin.

Eine bevorzugte Gruppe von Fluorchinolonen sind die der Formel (I) oder (II): 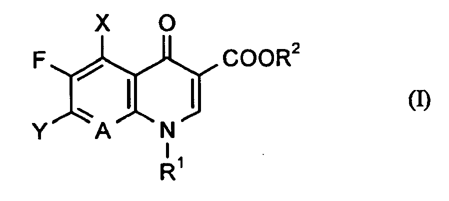 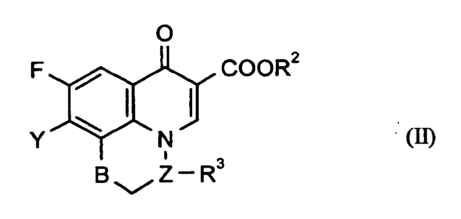 in welchen
- X: für Wasserstoff, Halogen, C₁₋₄-Alkyl, C₁₋₄-Alkoxy, NH₂ steht,
- Y: für Reste der Strukturen 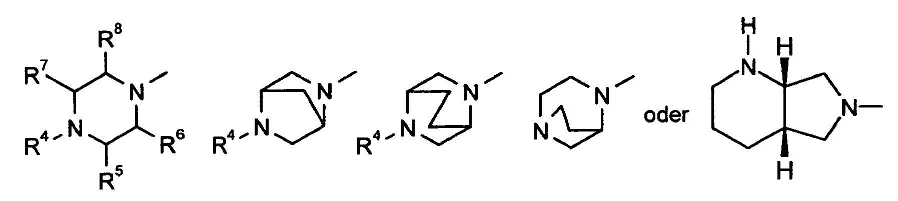
steht, worin
- R⁴: für gegebenenfalls durch Hydroxy oder Methoxy substituiertes geradkettiges oder verzweigtes C₁-C₄-Alkyl, Cyclopropyl, Acyl mit 1 bis 3 C-Atomen steht,
- R⁵: für Wasserstoff, Methyl, Phenyl, Thienyl oder Pyridyl steht,
- R⁶: für Wasserstoff oder C₁₋₄-Alkyl steht,
- R⁷: für Wasserstoff oder C₁₋₄-Alkyl steht,
- R⁸: für Wasserstoff oder C₁₋₄-Alkyl steht,
sowie
- R¹: für einen Alkylrest mit 1 bis 3 Kohlenstoffatomen, Cyclopropyl, 2-Fluorethyl, Methoxy, 4-Fluorphenyl, 2,4-Difluorphenyl oder Methylamino steht,
- R²: für Wasserstoff oder gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht,
- R³: für Wasserstoff, Methyl oder Ethyl steht und
- A: für Stickstoff, =CH-, =C(Halogen)-, =C(OCH₃)-, =C(CH₃)- oder =C(CN) steht,
- B: für Sauerstoff, gegebenenfalls durch Methyl oder Phenyl substituiertes =NH oder =CH₂ steht,
- Z: für =CH- oder =N- steht,
und deren pharmazeutisch verwendbaren Salze und Hydrate.

Die Verbindungen der Formeln (I) und (II) können in Form ihrer Racemate oder in enantiomeren Formen vorliegen.

Bevorzugt sind Verbindungen der Formel (I),
in welcher
- A: für =CH- oder =C-CN steht,
- R¹: für gegebenenfalls durch Halogen substituiertes C₁-C₃-Alkyl oder Cyclopropyl steht,
- R²: für Wasserstoff oder C₁₋₄-Alkyl steht,
- Y: für Reste der Strukturen 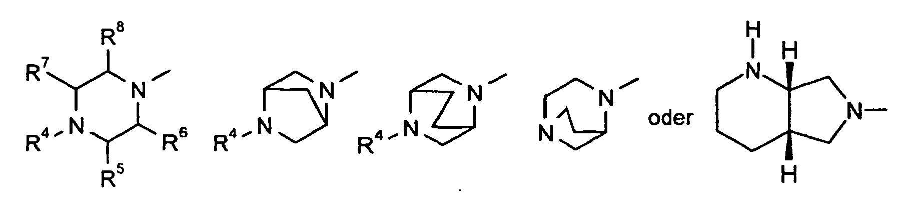
steht, worin
- R⁴: für gegebenenfalls durch Hydroxy substituiertes geradkettiges oder verzweigtes C₁-C₃-Alkyl, Oxoalkyl mit 1 bis 4 C-Atomen steht,
- R⁵: für Wasserstoff, Methyl oder Phenyl steht,
- R⁷: für Wasserstoff oder Methyl steht,
und deren pharmazeutisch verwendbaren Hydrate und Salze.

Besonders bevorzugt sind Verbindungen der Formel (I),
in welcher
- A: für =CH- oder =C-CN steht,
- R¹: für Cyclopropyl steht,
- R²: für Wasserstoff, Methyl oder Ethyl steht,
- Y: für Reste der Strukturen 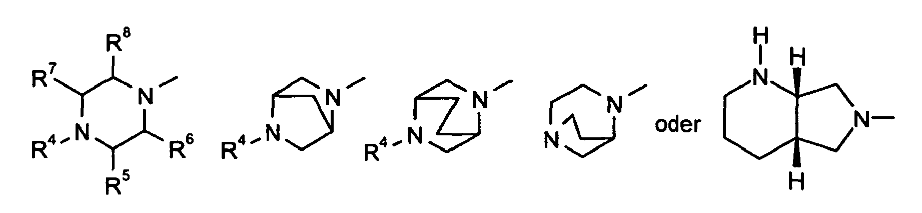
steht, worin
- R⁴: für Methyl, gegebenenfalls durch Hydroxy substituiertes Ethyl steht,
- R⁵: für Wasserstoff oder Methyl steht,
- R⁷: für Wasserstoff oder Methyl steht,
und deren pharmazeutisch verwendbaren Salze und Hydrate.

Als Salze kommen pharmazeutisch verwendbare Säureadditionssalze und basische Salze in Frage.

Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen. Ferner lassen sich die erfindungsgemäßen Verbindungen an saure oder basische Ionenaustauscher binden. Als pharmazeutisch verwendbare basische Salze seien die Alkalisalze, beispielsweise die Natrium- oder Kaliumsalze, die Erdalkalisalze, beispielsweise die Magnesium-, oder Calciumsalze; die Zinksalze, die Silbersalze und die Guanidiniumsalze genannt.

Unter Hydraten werden sowohl die Hydrate der Fluorchinolone selbst als auch die Hydrate von deren Salzen verstanden.

Als besonders bevorzugte Fluorchinolone seien die in WO 97/31001 beschriebenen Verbindungen genannt, insbesondere 8-Cyan-1-cyclopropyl-7-((1S,6S)-2,8-diazabicyclo[4.3.0]nonan-8-yl)-6-fluor-1,4-dihydro-4-oxo-3-chinolincarbonsäure (Pradofloxacin) mit der Formel

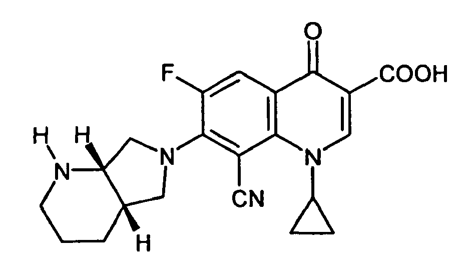

Weiterhin besonders bevorzugt eingesetzt wird Enrofloxacin:
1-Cyclopropyl-7-(4-ethyl-1-piperazinyl)-6-fluor-1,4-dihydro-4-oxo-3-chinolincarbonsäure

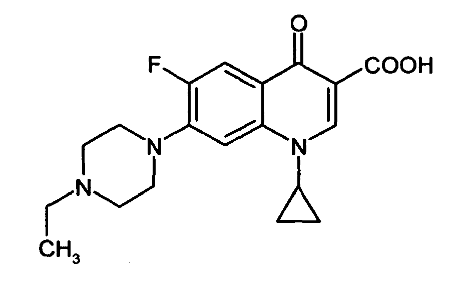

Weiterhin als Wirkstoff bevorzugt ist das Analgetikum Flupirtin der folgenden Formel:

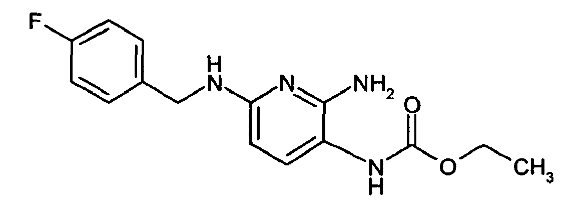

Flupirtin kann ebenfalls in Form seiner pharmazeutisch verwendbaren Salze eingesetzt werden, bevorzugt Salze mit Säuren, wie zum Beispiel das Hydrochlorid oder das Maleat.

Der pharmazeutisch wirksame Bestandteil wird üblicherweise in einem Anteil von 0,1 bis 20 Gew.-%, bevorzugt von 0,1 bis 15 Gew.-%, insbesondere von 1 bis 15 Gew.-% bezogen auf das Gesamtgewicht des Arzneimittels eingesetzt. Für Nutztiere sind relativ hohe Konzentrationen von 1 bis 15 Gew.-%, bevorzugt 5 bis 15 Gew.-% bezogen auf das Gesamtgewicht des Arzneimittels üblich. Bei Hobbytieren, wie z. B. Hunden und Katzen sind etwas niedrigere Konzentrationen im Bereich 0,1 bis 12 Gew.-%, bevorzugt 1 bis 6 Gew.-% bezogen auf das Gesamtgewicht des Arzneimittels üblich.

Als Kosolventien, die die Viskosität der Formulierungen herabsetzen, seien beispielsweise genannt: Pharmazeutisch verträgliche Alkohole, Dimethylsulfoxid, Ethyllactat, Ethylacetat, Triacetin, N-Methylpyrrolidon, Propylencarbonat, Glycofurol, Dimethylacetamid, 2-Pyrrolidon, Glycerin und Polyethylenglykole.

Die erfindungsgemäßen Arzneimittel enthalten als Kosolventien pharmazeutisch verträgliche Alkohole, wie z.B. Ethanol, Benzylalkohol oder n-Butanol. Diese werden üblicherweise in Anteilen von 1 bis 10 Gew.-%, bevorzugt von 3 bis 8 Gew.-% eingesetzt. Auch Mischungen der vorstehend genannten Lösungsmittel können als Kosolvenz eingesetzt werden.

Die erfindungsgemäßen Arzneimittel zeichnen sich durch gute Löslichkeitseigenschaften für Wirkstoffe aus, so dass in vielen Fällen auch mit der neutralen Form des Wirkstoffs (z.B. zwitterionische Form bei den Chinolonantibiotika) ausreichende Wirkstoffkonzentrationen in dem Arzneimittel erreicht werden. So können in Abhängigkeit von dem Wirkstoff und seinem isoelektrischen Punkt Mittel hergestellt werden, deren pH-Wert vorteilhafterweise in der Nähe des Neutralpunktes liegt, vorzugsweise bei pH 6 bis 8. Üblicherweise werden bei Arzneimitteln, z. B. bei Injektionsformulierungen, extreme pH-Werte vermieden, da die Verträglichkeiten und Stabilitäten in der Nähe von pH 7 in der Regel besser sind. Als Beispiel seien erfindungsgemäße Formulierungen des Pradofloxacin genannt, in denen das Pradofloxacin bei pH 7,4 in ausreichendem Maße löslich ist. Der pH-Wert wird eingestellt, indem man den erfindungsgemäßen Mitteln pharmazeutisch annehmbare Säuren oder Basen, z.B. als wässrige Lösung, zusetzt.

Die erfindungsgemäßen Arzneimittel können hergestellt werden, indem man das Solvens mit SAIB mischt und den Wirkstoff in der Mischung gelöst. Kosolventien sowie weitere Inhaltsstoffe können bereits dem Solvens zugegeben oder später zugemischt werden.

Alternativ kann der Wirkstoff zunächst im Solvens gelöst werden, dieser Lösung fügt man dann ggf. weitere Kosolventien zu und gibt schließlich das SAIB zu.

Die erfindungsgemäßen pharmazeutischen Zubereitungen eignen sich generell für die Anwendung bei Mensch und Tier. Bevorzugt werden sie in der Tierhaltung und Tierzucht bei Nutz-, Zucht-, Zoo-, Labor-, Versuchs- und Hobbytieren eingesetzt.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär sowie Vögel wie z.B. Hühner, Gänse, Puten, Enten, Tauben und Vogelarten für Heim- und Zoohaltung.

Zu Labor- und Versuchstieren gehören Mäuse, Ratten, Meerschweinchen, Goldhamster, Hunde und Katzen.

Zu den Hobbytieren gehören Kaninchen, Hamster, Meerschweinchen, Mäuse, Pferde, Reptilien, entsprechende Vogelarten, Hunde und Katzen.

Weiterhin seien Fische genannt, und zwar Nutz-, Zucht-, Aquarien- und Zierfische aller Altersstufen, die in Süß- und Salzwasser leben.

Bevorzugt werden die erfindungsgemäßen Zubereitungen bei Hobbytieren wie Pferden, Katzen und Hunden eingesetzt. Insbesondere eignen sie sich für die Anwendung bei Katzen und Hunden.

Beispiele für bevorzugte Nutztiere sind Rind, Schaf, Schwein und Huhn.

Die Anwendung kann sowohl prophylaktisch als auch therapeutisch erfolgen.

Die hier beschriebenen Formulierungen können auf verschiedene Arten dem Zielorganismus (Mensch oder Tier) zugeführt werden. Sie können beispielsweise parenteral (z.B. subcutan, intramuskulär, intraperitoneal), dermal, oral, rectal, vaginal oder nasal appliziert werden, wobei die parenterale Applikation bevorzugt ist.

Bevorzugt werden die Formulierungen als Lösungen, Paste, Suspension oder Emulsion gegeben. Sofern eine Lösung injiziert wird, diffundiert die geringe Menge des Lösungsmittels in das umliegende Gewebe oder die interstitielle Flüssigkeit und hinterlässt ein hochviskoses Depot, aus dem der Wirkstoff verzögert freigesetzt wird. Durch Zusatz eines Additivs, wie z.B. eines Polymers kann die Abbaugeschwindigkeit des Depots verlangsamt werden.

Als biologisch abbaubare Polymere oder Oligomere seien beispielsweise genannt: Poly(lactide), Poly(lactid-coglycolide), Poly(glycolide), Poly(caprolactone), Polyamide, Polyanhydride, Polyaminosäuren, Polyorthoester, Polycyanacrylate, Poly(phosphazine), Poly(phosphorester), Polyesteramide, Polydioxanone, Polyacetale, Polyketale, Polycarbonate, Polyorthocarbonate, abbaubare Polyurethane, Polyhydroxybutyrate, Polyhydroxyvalerate, Polyalkylenoxalate, Polyalkylensuccinate, Poly(äpfelsäure), Chitin, Chitosan, sowie Copolymere, Terpolymere, oxidierte Cellulose oder Kombinationen oder Mischungen der vorstehend genannten Materialien. Beispiele für Poly-(alpha-hydroxysäuren) sind unter anderem: Poly(glycolsäure), Poly(DL-Milchsäure) und Poly(L-Milchsäure) und deren Copolymere. Beispiele für Polylactone sind unter anderem: Poly(epsilon-caprolacton), Poly(delta-valerolacton) und Poly(gamma-butyrolacton).

Als nicht biologisch abbaubare Polymere oder Oligomere seien beispielsweise genannt: Polyacrylate, Ethylen-Vinylacetat-Polymere, Cellulose und Cellulose-Derivate, Acyl-substituierte Celluloseacetate und Derivate davon, nicht-abbaubare Polyurethane, Polystyrole, Polyvinylchlorid, Polyvinylfluorid, Polyvinylimidazol, chlorsulfonierte Polyolefine und Polyethylenoxid; davon bevorzugt sind beispielsweise: Polyethylen, Polyvinylpyrrolidon, Ethylen-Vinylacetat, Polyethylenglycol, Celluloseacetatbutyrat ("CAB") und Celluloseacetatpropionat ("CAP").

Je nach Art der Formulierung und Applikationsform können die erfindungsgemäßen Arzneimittel weitere übliche, pharmazeutisch verträgliche Zusatz- und Hilfsstoffe enthalten. Als Beispiele seien genannt
- Konservierungsstoffe wie zum Beispiel Carbonsäuren (Sorbinsäure, Propionsäure, Benzoesäure, Milchsäure), Phenole (Kresole, p-Hydroxybenzoesäureester wie Methylparaben, Propylparaben etc.), aliphatische Alkohole (Benzylalkohol, Ethanol, Butanol etc.), quartäre Ammoniumverbindungen (Benzalkoniumchlorid, Cetylpyridiniumchlorid)
- Antioxidantien wie zum Beispiel Sulfite (Na-sulfit, Na-metabisulfit), organische Sulfide (Cystin, Cystein, Cysteamin, Methionin, Thioglycerol, Thioglykolsäure, Thiomilchsäure) Phenole (Tocopherole, wie auch Vitamin E und Vitamin-E-TPGS (d-alpha-Tocopherylpolyethylenglykol-1000-succinat), Butylhydroxyanisol, Butylhydroxytoluol, Octyl- und Dodecylgallat), organische Säuren (Ascorbinsäure, Citronensäure, Weinsäure, Milchsäure) und deren Salze und Ester
- Netzmittel wie zum Beispiel Fettsäuresalze, Fettalkylsulfate, Fettalkylsulfonate, lineare Alkylbenzolsulfonate, Fettalkylpolyethylen-glykolethersulfate, Fettalkylpolyethylenglykolether, Alkylphenolpolyethylenglykolether, Alkylpolyglykoside, Fettsäure-N-methylglucamide, Polysorbate, Sorbitanfettsäureester und Poloxamere.
- Pharmazeutisch akzeptable Farbstoffe wie zum Beispiel Eisenoxide, Carotinoide, etc.

Die Verwendung von SAIB in Kombination mit den genannten Solventien führt zu Arzneimitteln mit guter Löslichkeit und Stabilität des Wirkstoffs. Weiterhin zeichnet sich das erfindungsgemäße Arzneimittel durch gute Verträglichkeit insbesondere nach parenteraler Gabe aus. Darüber hinaus lässt sich die Wirkstofffreisetzung beeinflussen.

### Beispiele

Die Formulierungen der folgenden Beispiele werden hergestellt, indem die Solventien bzw. Kosolventien mit SAIB gemischt und die verschiedenen Wirkstoffe (Pradofloxacin, Enrofloxacin, Flupirtin) in den Mischungen gelöst werden. Der pH-Wert der Lösungen kann durch Zugabe von Säuren oder Basen eingestellt werden. Es resultieren klare Lösungen von mittlerer Viskosität. Die Lösungen werden sterilfiltriert und in geeignete Behältnisse überführt. (Prozentangaben in Gewichtsprozent bezogen auf das Gesamtgewicht des fertigen Präparats).

### Beispiel 1

40 % SAIB
3 % Pradofloxacin
3 % n-Butanol
5 % Ethanol
1,7 % IN-HCl
ad 100 % Glycerinformal
3 g Pradofloxacin werden in einem Gemisch aus 3 g n-Butanol, 5 g Ethanol, 40 g SAIB, und 47,3 g Glycerinformal gelöst. Zum Einstellen des pH-Wertes auf ca. 7,4 werden 1,7 g IN-Salzsäure zu der Lösung gegeben.

### Beispiel 2

40 % SAIB
1 % Enrofloxacin
5 % Ethanol
ad 100 % Solketal
1 g Enrofloxacin werden in einem Gemisch aus 40 g SAIB, 5 g Ethanol und 54 g Solketal gelöst.

### Beispiel 3

30 % SAIB
1,5 % Pradofloxacin
3 % n-Butanol
ad 100 % Solketal
1,5 g Pradofloxacin werden in einem Gemisch aus 3 g n-Butanol, 30 g SAIB und 65,5 g Solketal gelöst.

### Beispiel 4

50 % SAIB
5 % Flupirtin
5 % Ethanol
ad 100 % Glycerinformal
5 g Flupirtin werden in einem Gemisch aus 5 g Ethanol, 50 g SAIB und 40 g Glycerinformal gelöst.

### Beispiel 5

10 % SAIB
6 % Pradofloxacin
5 % Ethanol
2,4 % 1N-HCl
ad 100 % Glycerinformal
6 g Pradofloxacin werden in einem Gemisch aus 5 g Ethanol, 10 g SAIB und 76,6 g Glycerinformal gelöst. Zum Einstellen des pH-Wertes auf ca. 7,4 werden 2,4 g 1N-Salzsäure zu der Lösung gegeben.

## Patentansprüche

1. Arzneimittel enthaltend:
(a) einen pharmazeutisch wirksamen Bestandteil
(b) Saccharoseacetatisobutyrat (SAIB)
(c) als Solvens Glycerinformal oder Isopropylidenglycerol oder ein Gemisch davon
sowie als Kosolvenz einen pharmazeutisch verträglichen Alkohol.

2. Arzneimittel gemäß Anspruch 1, enthaltend als Kosolvenz Ethanol, n-Butanol oder Benzylalkohol.

3. Arzneimittel gemäß Anspruch 1 oder 2, enthaltend SAIB in einer Menge von maximal 80 Gew.-%, bezogen auf das Gesamtgewicht der Formulierung.

4. Arzneimittel gemäß einem der vorstehenden Ansprüche, enthaltend ein Fluorchinolon der Formel (I) oder (II): 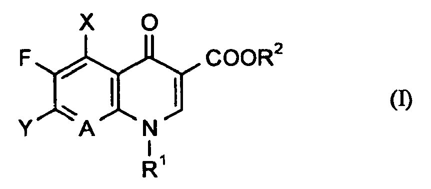 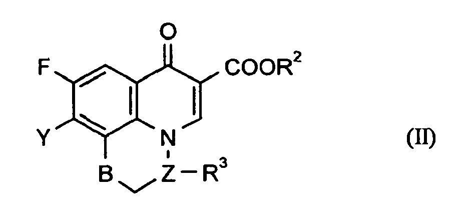 in welchen
X für Wasserstoff, Halogen, C₁₋₄-Alkyl, C₁₋₄-Alkoxy, NH₂ steht,
Y für Reste der Strukturen 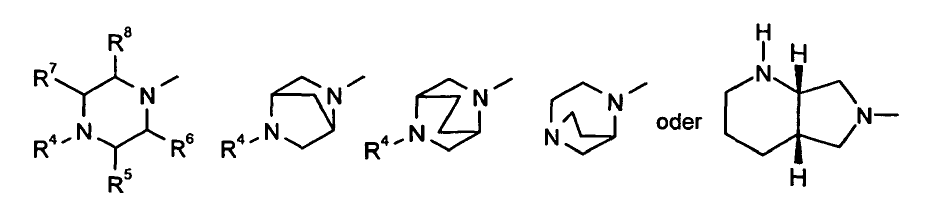
steht, worin
R⁴ für gegebenenfalls durch Hydroxy oder Methoxy substituiertes geradkettiges oder verzweigtes C₁-C₄-Alkyl, Cyclopropyl, Acyl mit 1 bis 3 C-Atomen steht,
R⁵ für Wasserstoff, Methyl, Phenyl, Thienyl oder Pyridyl steht,
R⁶ für Wasserstoff oder C₁₋₄-Alkyl steht,
R⁷ für Wasserstoff oder C₁₋₄-Alkyl steht,
R⁸ für Wasserstoff oder C₁₋₄-Alkyl steht,
sowie
R¹ für einen Alkylrest mit 1 bis 3 Kohlenstoffatomen, Cyclopropyl, 2-Fluorethyl, Methoxy, 4-Fluorphenyl, 2,4-Difluorphenyl oder Methylamino steht,
R² für Wasserstoff oder gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht,
R³ für Wasserstoff, Methyl oder Ethyl steht und
A für Stickstoff, =CH-, =C(Halogen)-, =C(OCH₃)-, =C(CH₃)- oder =C(CN) steht,
B für Sauerstoff, gegebenenfalls durch Methyl oder Phenyl substituiertes =NH oder =CH₂ steht,
Z für =CH- oder =N- steht,
und deren pharmazeutisch verwendbaren Salze und Hydrate.

5. Arzneimittel gemäß Anspruch 4 enthaltend ein Fluorchinolon der Formel (I),
in welcher
A für =CH- oder =C-CN steht,
R¹ für gegebenenfalls durch Halogen substituiertes C₁-C₃-Alkyl oder Cyclopropyl steht,
R² für Wasserstoff oder C₁₋₄-Alkyl steht,
Y für Reste der Strukturen 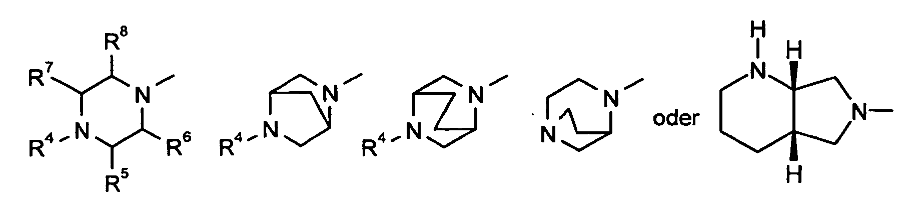
steht, worin
R⁴ für gegebenenfalls durch Hydroxy substituiertes geradkettiges oder verzweigtes C₁-C₃-Alkyl, Oxoalkyl mit 1 bis 4 C-Atomen steht,
R⁵ für Wasserstoff, Methyl oder Phenyl steht,
R⁷ für Wasserstoff oder Methyl steht,
und deren pharmazeutisch verwendbaren Hydrate und Salze.

6. Arzneimittel gemäß Anspruch 4 enthaltend ein Fluorchinolon der Formel (I),
in welcher
A für =CH- oder =C-CN steht,
R¹ für Cyclopropyl steht,
R² für Wasserstoff, Methyl oder Ethyl steht,
Y für Reste der Strukturen 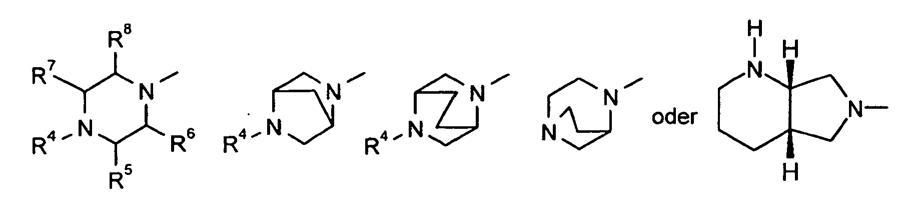
steht, worin
R⁴ für Methyl, gegebenenfalls durch Hydroxy substituiertes Ethyl steht,
R⁵ für Wasserstoff oder Methyl steht,
R⁷ für Wasserstoff oder Methyl steht,
und deren pharmazeutisch verwendbaren Salze und Hydrate.

7. Arzneimittel gemäß Anspruch 4, enthaltend Enrofloxacin.

8. Arzneimittel gemäß Anspruch 4, enthaltend Pradofloxacin.

9. Arzneimittel gemäß einem der Ansprüche 1 bis 3, enthaltend Flupirtin.

## Claims

1. Medicament comprising:
(a) a pharmaceutically active constituent
(b) sucrose acetate isobutyrate (SAIB)
(c) as solvent glycerol formal or isopropylideneglycerol or a mixture thereof,
and as cosolvent a pharmaceutically acceptable alcohol.

2. Medicament according to Claim 1, comprising as cosolvent ethanol, n-butanol or benzyl alcohol.

3. Medicament according to Claim 1 or 2, comprising SAIB in an amount not exceeding 80% by weight based on the total weight of the formulation.

4. Medicament according to any of the preceding claims, comprising a fluoroquinolone of the formula (I) or (II): 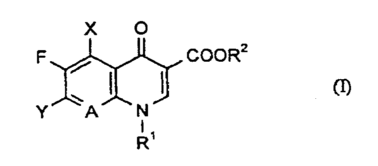 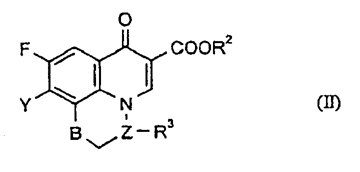 in which
X is hydrogen, halogen, C₁₋₄-alkyl, C₁₋₄-alkoxy, NH₂,
Y is radicals of the structures 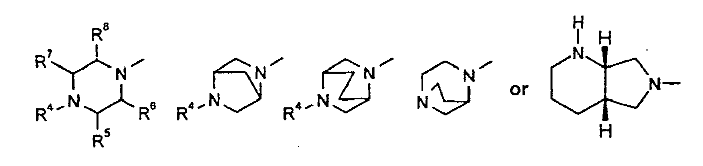
in which
R⁴ is optionally hydroxy- or methoxy-substituted straight-chain or branched C₁-C₄-alkyl, cyclopropyl, acyl having 1 to 3 C atoms,
R⁵ is hydrogen, methyl, phenyl, thienyl or pyridyl,
R⁶ is hydrogen or C₁₋₄-alkyl,
R⁷ is hydrogen or C₁₋₄-alkyl,
R⁸ is hydrogen or C₁₋₄-alkyl,
and
R¹ is an alkyl radical having 1 to 3 carbon atoms, cyclopropyl, 2-fluoroethyl, methoxy, 4-fluorophenyl, 2,4-difluorophenyl or methylamino,
R² is hydrogen or optionally methoxy- or 2-methoxyethoxy-substituted alkyl having 1 to 6 carbon atoms, and cyclohexyl, benzyl, 2-oxopropyl, phenacyl, ethoxycarbonylmethyl, pivaloyloxymethyl,
R³ is hydrogen, methyl or ethyl, and
A is nitrogen, =CH-, =C(halogen)-, =C(OCH₃)-, =C(CH₃)- or =C(CN),
B is oxygen, optionally methyl- or phenyl-substituted =NH or =CH₂,
Z is =CH- or =N-,
and the pharmaceutically useable salts and hydrates thereof.

5. Medicament according to Claim 4 comprising a fluoroquinolone of the formula (I)
in which
A is =CH- or =C-CN,
R¹ is optionally halogen-substituted C₁-C₃-alkyl or cyclopropyl,
R² is hydrogen or C₁₋₄-alkyl,
Y is radicals of the structures 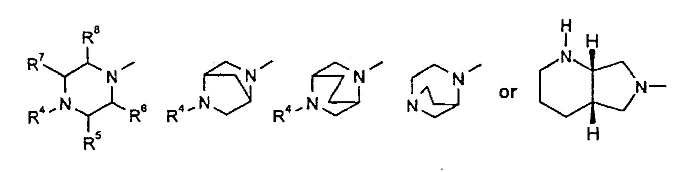
in which
R⁴ is optionally hydroxy-substituted straight-chain or branched C₁-C₃-alkyl, oxoalkyl having 1 to 4 C atoms,
R⁵ is hydrogen, methyl, or phenyl,
R⁷ is hydrogen or methyl,
and the pharmaceutically useable hydrates and salts thereof.

6. Medicament according to Claim 4 comprising a fluoroquinolone of the formula (I),
in which
A is =CH- or =C-CN,
R¹ is cyclopropyl,
R² is hydrogen, methyl or ethyl,
Y is radicals of the structures 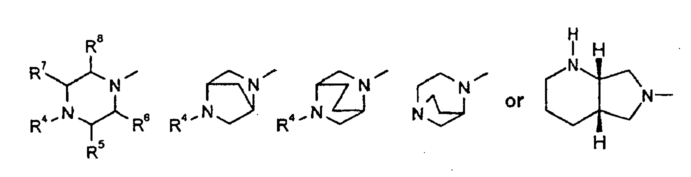
in which
R⁴ is methyl, optionally hydroxy-substituted ethyl,
R⁵ is hydrogen or methyl,
R⁷ is hydrogen or methyl,
and the pharmaceutically useable salts and hydrates thereof.

7. Medicament according to Claim 4, comprising enrofloxacin.

8. Medicament according to Claim 4, comprising pradofloxacin.

9. Medicament according to any of Claims 1 to 3, comprising flupirtine.

## Revendications

1. Médicament contenant :
(a) un constituant pharmaceutiquement actif
(b) de l'acétate-isobutyrate de saccharose (SAIB)
(c) comme solvant, du formal de glycérol ou de l'isopropylidèneglycérol ou un mélange des deux ainsi que, comme cosolvant, un alcool pharmaceutiquement compatible.

2. Médicament suivant la revendication 1, contenant comme cosolvant de l'éthanol, du n-butanol ou de l'alcool benzylique.

3. Médicament suivant la revendication 1 ou 2, contenant du SAIB en une quantité maximale égale à 80 % en poids, par rapport au poids total de la formulation.

4. Médicament suivant l'une des revendications précédentes, contenant une fluoroquinolone de formule (I) ou (II) : 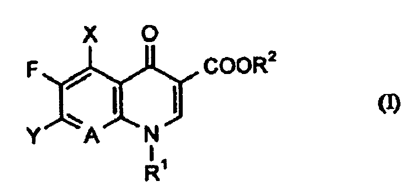 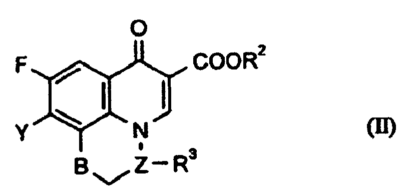 formules dans lesquelles
X représente l'hydrogène, un halogène, un reste alkyle en C₁ à C₄, alkoxy en C₁ à C₄, un groupe NH₂,
Y représente des restes de formules 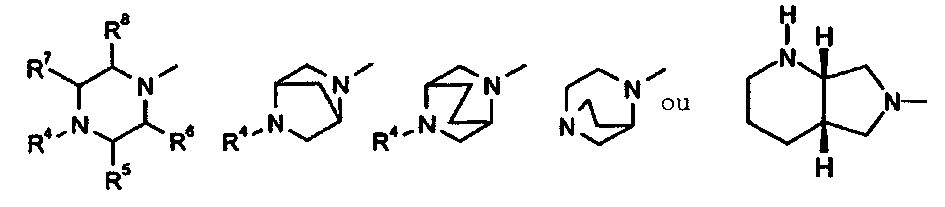
où
R⁴ désigne un reste alkyle en C₁ à C₄ linéaire ou ramifié éventuellement substitué par un radical hydroxy ou méthoxy, un reste cyclopropyle, acyle ayant 1 à 3 atomes de carbone,
R⁵ représente l'hydrogène, un reste méthyle, phényle, thiényle ou pyridyle,
R⁶ représente l'hydrogène ou un reste alkyle en C₁ à C₄,
R⁷ représente l'hydrogène ou un reste alkyle en C₁ à C₄,
R⁸ représente l'hydrogène ou un reste alkyle en C₁ à C₄,
de même que
R¹ représente un reste alkyle ayant 1 à 3 atomes de carbone, cyclopropyle, 2-fluoréthyle, méthoxy, 4-fluorophényle, 2,4-difluorophényle ou méthylamino,
R² représente l'hydrogène ou un reste alkyle ayant 1 à 6 atomes de carbone, éventuellement substitué par un radical méthoxy ou 2-méthoxyéthoxy, ainsi qu'un reste cyclohexyle, benzyle, 2-oxopropyle, phénacyle, éthoxycarbonylméthyle, pivaloyloxyméthyle,
R³ représente l'hydrogène, un reste méthyle ou éthyle, et
A représente l'azote, un groupe =CH-, =C(halogéno)-, =C(OCH₃)-, =C(CH₃)- ou =C(CN),
B représente l'oxygène, un groupe =NH éventuellement substitué par un radical méthyle ou phényle ou un groupe =CH₂,
Z représente un groupe =CH- ou =N-,
ainsi que leurs sels et leurs hydrates pharmaceutiquement acceptables.

5. Médicament suivant la revendication 4, contenant une fluoroquinolone de formule (I) :
dans laquelle
A représente un groupe =CH- ou =C-CN,
R¹ représente un reste alkyle en C₁ à C₃ éventuellement substitué par un halogène ou un reste cyclopropyle,
R² représente l'hydrogène ou un reste alkyle en C₁ à C₄,
Y représente des restes de formules 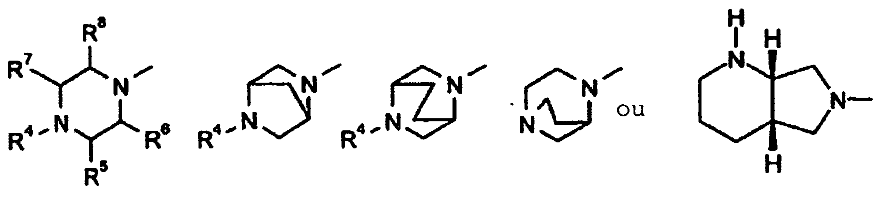
où
R⁴ représente un reste alkyle en C₁ à C₃ linéaire ou ramifié éventuellement substitué par un radical hydroxy, un reste oxoalkyle ayant 1 à 4 atomes de carbone,
R⁵ représente l'hydrogène, un reste méthyle ou phényle,
R⁷ représente l'hydrogène ou un reste méthyle,
ainsi que leurs hydrates et leurs sels pharmaceutiquement acceptables.

6. Médicament suivant la revendication 4, contenant une fluoroquinolone de formule (I) :
dans laquelle
A représente un groupe =CH- ou =C-CN,
R¹ représente un reste cyclopropyle,
R² représente l'hydrogène, un reste méthyle ou éthyle,
Y représente des restes de formules 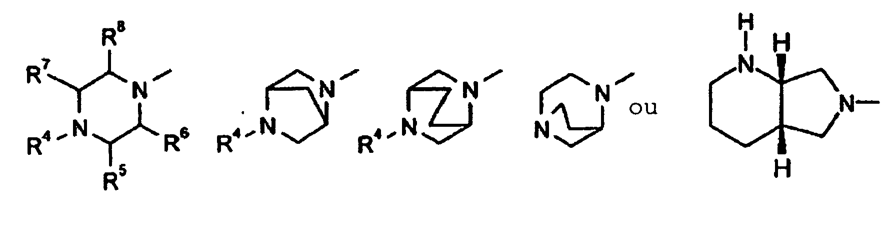
où
R⁴ représente un reste méthyle, un reste éthyle éventuellement substitué par un radical hydroxy,
R⁵ représente l'hydrogène ou un reste méthyle,
R⁷ représente l'hydrogène ou un reste méthyle,
et leurs sels et hydrates pharmaceutiquement acceptables.

7. Médicament suivant la revendication 4, contenant de l'enrofloxacine.

8. Médicament suivant la revendication 4, contenant de la pradofloxacine.

9. Médicament suivant l'une des revendications 1 à 3, contenant de la flupirtine.
